# EUROPEAN PATENT APPLICATION

(11) **EP 4 550 247 A1**
(43) Date of publication of application: **07.05.2025**
(21) Application number: 23831433.0
(22) Date of filing: 27.06.2023
(51) Int. Cl.: G06Q 50/10

(54) **INFORMATION PROCESSING METHOD, INFORMATION PROCESSING SYSTEM, INFORMATION PROCESSING DEVICE, AND PROGRAM**

(30) Priority: 28.06.2022 JP 2022103414
(71) Applicant: Revorn Co., Ltd., Tokyo 104-0033 (JP)
(72) Inventor: MATSUOKA, Hiroaki, Tokyo 104-0033 (JP)
(74) Representative: Winter, Brandl - Partnerschaft mbB
(86) International application number: PCT/JP2023/023742
(87) International publication number: WO 2024/004993

(57) **Abstract**

[Problem] To provide an information processing method, an information processing system, an information processing device, and a program that allow a person to easily recognize a result of determining or identifying a smell.

[Solution] According to an aspect of the present invention, an information processing method performed by an information processing system is provided. This information processing method includes a first acquisition step, a determination step, and a presentation step. In the first acquisition step, target data is acquired. The target data is smell data detected by a sensor from an evaluation target. In the determination step, a correlation between the target data and at least part of reference data is determined. The reference data is smell data based on a plurality of predefined reference smells. In the presentation step, smell information is presented. The smell information is information obtained by visualizing, on the basis of the correlation, the target data by using, as an index, reference data for which the correlation has been determined.

## Description

### BACKGROUND

The present disclosure relates to an information processing method, an information processing system, an information processing apparatus, and a program.

### RELATED ART

In general, an odor is not something that can be confirmed visually, but techniques have been proposed to visualize it (see, for example, Patent Document 1). Furthermore, techniques for identifying objects by an odor have also been proposed (see, for example, Patent Document 2).

### PRIOR ART DOCUMENTS

### Patent document

[Patent Document 1] JP2021-76458 A
[Patent document 2] WO2020/116490

### SUMMARY

### Problems to be Solved by Invention

By the way, when visualizing an odor, the corresponding odor may be expressed in words. In this case, it is required to accurately express the odor by limiting the words used to a certain range.

In view of the above circumstances, the present disclosure provides an information processing method, an information processing system, an information processing apparatus, and a program, which allow a person to easily recognize a result of an odor determination and identification.

### Means for Solving Problems

According to an aspect of the present disclosure, an information processing method executed by an information processing system is provided. The information processing method comprises a first acquisition step, a specification step, and a presentation step. The first acquisition step acquires target data. The target data is odor data detected from an evaluation target by a sensor. The specification step specifies a correlation between the target data and at least a part of reference data. The reference data is odor data based on a plurality of predetermined reference odors. The presentation step presents odor information. The odor information is information that visualizes the target data based on the correlation, using the reference data that has specified the correlation as the index.

According to one aspect of the present disclosure, it is possible to visually recognize an odor.

### BRIEF DESCRIPTION OF DRAWINGS

[FIG. 1] FIG. 1 shows an example of a configuration of a system 100 according to an embodiment of the present disclosure.
[FIG. 2] FIG. 2 shows a configuration of an information processing apparatus 1.
[FIG. 3] FIG. 3 shows a configuration of an information processing terminal 3.
[FIG. 4] FIG. 4 shows a configuration of a detection apparatus 4.
[FIG. 5] FIG. 5 shows a configuration of a detection apparatus 5.
[FIG. 6] FIG. 6 is a block diagram showing a functional configuration of the information processing apparatus 1.
[FIG. 7] FIG. 7 is an activity diagram showing a flow of generating reference data.
[FIG. 8] FIG. 8 shows an example of an aroma wheel.
[FIG. 9] FIG. 9 shows an example of reference odors used for evaluation by an appraiser.
[FIG. 10] FIG. 10 shows an example of evaluation by an appraiser.
[FIG. 11] FIG. 11 shows an example of evaluation by an appraiser.
[FIG. 12] FIG. 12 shows an example of evaluation by an appraiser.
[FIG. 13] FIG. 13 is an activity diagram showing a flow of presenting odor information.
[FIG. 14] FIG. 14 shows an example in which odor information is presented by a radar chart.
[FIG. 15] FIG. 15 shows an example in which odor information is presented using a circle.
[FIG. 16] FIG. 16 shows an example in which odor information is presented using objects.
[FIG. 17] FIG. 17 shows an example in which odor information is presented using an illustration image.
[FIG. 18] FIG. 18 shows an example of presentation information that visualizes a change over time of an odor.
[FIG. 19] FIG. 19 shows an example of presentation information that visualizes a change over time of an odor.
[FIG. 20] FIG. 20 shows an example of presentation information that visualizes a change over time of an odor.

### DETAILED DESCRIPTION

Hereinafter, an embodiment of the present disclosure will be described with reference to drawings. Various features described in the embodiment below can be combined with each other.

A program for realizing a software in the present embodiment may be provided as a non-transitory computer readable storage medium that can be read by a computer, may be provided for download from an external server, or may be provided in such a manner that the program can be activated on an external computer to realize function thereof on a client terminal (so-called cloud computing).

A term "unit" in the present embodiment may include, for example, a combination of a hardware resource implemented as circuits in a broad sense and information processing of software that can be concretely realized by the hardware resource. Furthermore, various kinds of information are described in the present embodiment, and such information may be represented by, for example, physical values of signal values representing voltage and current, high and low signal values as a set of binary bits consisting of 0 or 1, or quantum superposition (so-called qubits), and communication and computation may be executed on a circuit in a broad sense.

The circuit in a broad sense is a circuit realized by at least properly combining a circuit, circuitry, a processor, a memory, and the like. In other words, a circuit includes an application specific integrated circuit (ASIC), a programmable logic device (e.g., simple programmable logic device (SPLD), a complex programmable logic device (CLPD), field programmable gate array (FPGA), and the like.

### 1. Overall configuration

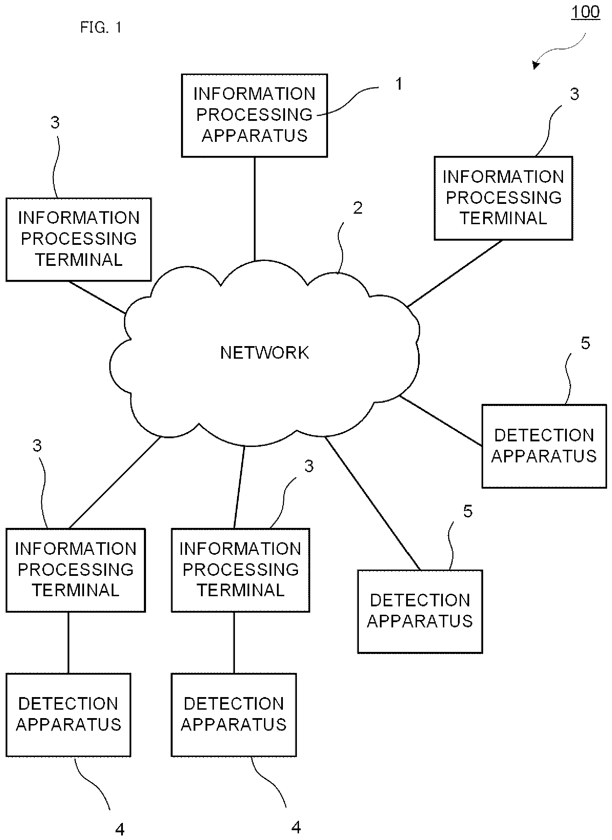

FIG. 1 shows an example of a configuration of a system 100 according to an embodiment of the present disclosure. As shown in the drawing, the system 100 is configured by connecting an information processing apparatus 1 to a network 2. An information processing terminal 3 is connected to the network 2, and a detection apparatus 4 is connected to a part of the information processing terminal 3. Furthermore, a detection apparatus 5 is connected to the network 2. There is no limit to the number of information processing terminals 3, the number of detection apparatuses 4, and the number of detection apparatuses 5, which are connected to the network 2, and only one of the information processing terminal 3 and the detection apparatus 5 needs to be connected to the network 2. The information processing terminal 3 and the detection apparatus 5 may be connected to the network 2 as necessary and need not be always connected.

The information processing apparatus 1 visualizes an odor detected by sensors included in the detection apparatus 4 and the detection apparatus 5, and an odor evaluated by a person, or the like. The network 2 enables communication between the information processing apparatus 1, the information processing terminal 3 and the detection apparatus 5, and includes, for instance, the Internet. The information processing terminal 3 is a tablet, a smartphone or a computer, communicates with the information processing apparatus 1, and controls the detection apparatus 4. The detection apparatus 4 includes a sensor and detects odors. Similar to the detection apparatus 4, the detection apparatus 5 is configured to detect odors and communicate with the information processing apparatus 1 without intervention of the information processing terminal 3.

### 2. Configuration of information processing apparatus 1

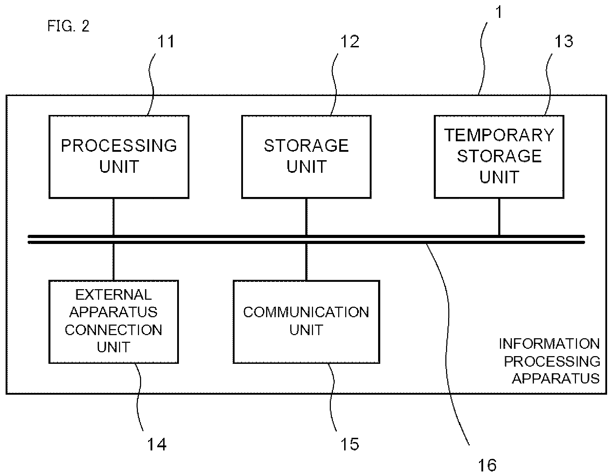

Next, a configuration of the information processing apparatus 1 will be described. FIG. 2 shows a configuration of the information processing apparatus 1. As shown in the drawing, the information processing apparatus 1 comprises a processing unit 11, a storage unit 12, a temporary storage unit 13, an external apparatus connection unit 14, and a communication unit 15, which are electrically connected inside the information processing apparatus 1 via a communication bus 16.

The processing unit 11 is realized by a central processing unit (CPU), for example, and operates in accordance with a predetermined program stored in the storage unit 12 to realize various functions.

The storage unit 12 is a nonvolatile storage medium that stores various information. This is realized, for example, by a storage device such as a hard disk drive (HDD) or a solid state drive (SSD). The storage unit 12 may be arranged in another apparatus communicable with the information processing apparatus 1.

The temporary storage unit 13 is a volatile storage medium. This is realized by a memory such as random access memory (RAM), for example, and temporarily stores necessary information (arguments, array, etc.) when the processing unit 11 operates.

The external apparatus connection unit 14 is a connection unit in compliance with standard such as universal serial bus (USB) or high-definition multimedia interface (HDMI (registered trademark)) and allows connection of an input apparatus such as the detection apparatus 4, a keyboard, etc., or a display device such as a monitor.

The communication unit 15 is a communication means in compliance with, for instance, a local area network (LAN) standard, and realizes communication between the information processing apparatus 1 and the network 2 such as a local area network or the Internet via the network.

The information processing apparatus 1 may be a computer or a personal computer for general purpose server. The information processing apparatus 1 may be configured by utilizing two or more computers, or may be configured as an information processing system.

### 3. Configuration of information processing terminal 3

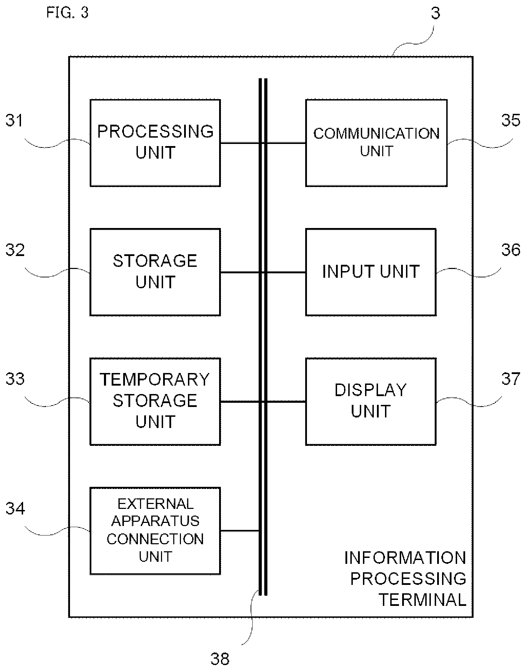

FIG. 3 shows a configuration of the information processing terminal 3. As shown in the drawing, the information processing terminal 3 comprises a processing unit 31, a storage unit 32, a temporary storage unit 33, an external apparatus connection unit 34, a communication unit 35, an input unit 36 and a display unit 37, which are electrically connected inside the information processing terminal 3 via a communication bus 38.

The processing unit 31 is realized by a CPU, for example, and operates in accordance with a predetermined program stored in the storage unit 32 to realize various functions.

The storage unit 32 is a nonvolatile storage medium that stores various information.

The temporary storage unit 33 is a volatile storage medium. This is realized by a memory such as a random access memory, for instance, so that necessary information (arguments, arrays, etc.) is temporarily stored when the processing unit 21 operates.

The external apparatus connection unit 34 is a connection unit in compliance with standard such as universal serial bus or Bluetooth (registered trademark), and enables the detection apparatus 4 and the like to be connected.

The communication unit 35 is a communication means in compliance with, for instance, a local area network (LAN) standard, and realizes communication between the information processing apparatus 1 and the network 2 such as a local area network or the Internet via the network. Further, the communication unit 35 also includes a communication means capable of communicating through a cellular phone network.

The input unit 36 is configured to receive an operation input. The display unit 37 is configured to display information or the like on a screen. The input unit 36 and the display unit 37 may be integrated as a touch panel.

A general-purpose smartphone, a tablet terminal, a notebook PC, etc. can be utilized for the information processing terminal 3.

### 4. Configuration of detection apparatus 4

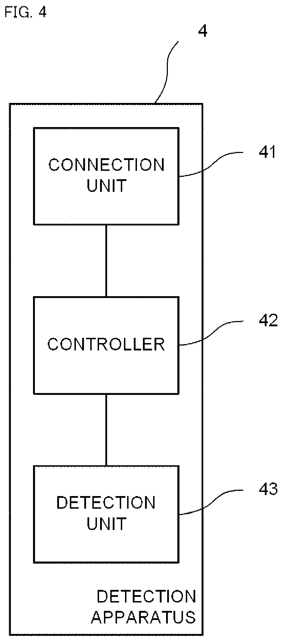

FIG. 4 shows a configuration of the detection apparatus 4. As shown in the drawing, the detection apparatus 4 comprises a connection unit 41, a controller 42, and a detection unit 43.

The connection unit 41 is in compliance with standard such as universal serial bus or Bluetooth (registered trademark), for instance, and enables the information processing terminal 3 and the like to be connected. The controller 42 controls operation of the detection unit 43 and transmits/receives information to/from the information processing terminal 3 and the like. The detection unit 43 includes a sensor that detects odors. The sensor included in the detection unit 43 is, for example, a sensor that detect gas such as carbon dioxide, carbon monoxide, methane, butane, ammonia, etc. or two or more Quartz Crystal Microbalance sensors, each of Quartz Crystal Microbalance sensors has a quartz crystal formed by a thin film having nonspecific adsorption properties, and a different compound is vapor-deposited on each of quartz crystals. The vapor-deposited compound is, for example, D-phenylalanine, D-tyrosine, DL-histidine, D-glucose, adenine, polyethylene, etc. For any one of these compounds, resonance frequency changes due to adhesion of odor component. Since the degree to which odor components adhere differs depending on the compound, each of the Quartz Crystal Microbalance sensors can detect a different odor.

### 5. Configuration of detection apparatus 5

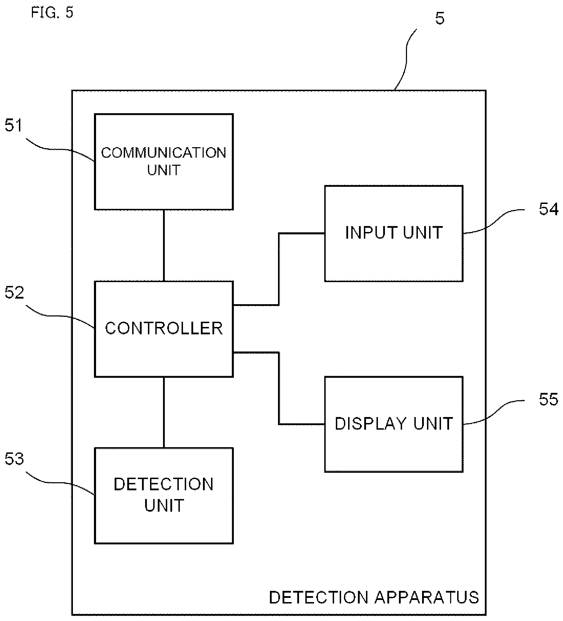

FIG. 5 shows a configuration of the detection apparatus 5. As shown in the drawing, the detection apparatus 5 comprises a communication unit 51, a controller 52, a detection unit 53, a display unit 55 and an input unit 54.

The communication unit 51 is a communication means in compliance with, for instance, a local area network (LAN) standard, and realizes communication between the detection apparatus 5 and the network 2 such as a local area network or the Internet via the network. Further, the communication unit 51 may include a communication means capable of communicating through a cellular phone network.

The controller 52 controls operation of the detection unit 53 and transmits/receives information to/from the information processing apparatus 1 or the like. The detection unit 53, same as the detection unit 43, includes a sensor that detects odors.

The input unit 54 is configured to receive an operation input. The display unit 55 is configured to display information or the like on the screen. The input unit 54 and the display unit 55 may be integrated as a touch panel.

### 6. Functional configuration of information processing apparatus 1

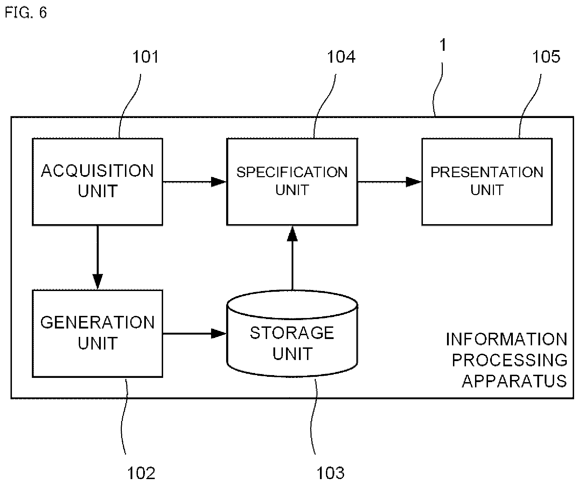

Next, the functions of the information processing apparatus 1 will be described. FIG. 6 is a block diagram showing a functional configuration of the information processing apparatus 1. As shown in the drawing, the information processing apparatus 1 comprises an acquisition unit 101, a generation unit 102, a storage unit 103, a specification unit 104, and a presentation unit 105. The acquisition unit 101 executes a first acquisition step and a second acquisition step. The generation unit 102 executes a generation step, and the storage unit 103 stores the reference data, evaluation conditions, etc. generated in the generation step. The specification unit 104 executes a specification step, and the presentation unit 105 executes a presentation step. The information processing apparatus 1 comprises at least one processor, for example, a processing unit 11, which is configured to execute a program so that an acquisition step, a specification step, and a presentation step, which are steps of the information processing method described later, are executed. As described above, the information processing apparatus 1 can be configured by a plurality of computers, and thus each functional unit shown in FIG. 6 can be configured to function on a separate computer. The information processing system in this case comprises at least one processor configured to execute a program so that each step of the information processing method is executed. Each of these functional units is realized by a program that allows the computer to function as the information processing apparatus 1, and the program causes at least one computer to execute each step of an information processing method described later.

### 7. Information processing method of information processing apparatus 1

Next, an information processing method executed by the information processing apparatus 1 will be described. This information processing method comprises a first acquisition step, a specification step, a presentation step, a second acquisition step, and a generation step. Here, the flow of information processing will be explained. This explanation will be divided into a generation of reference data and an evaluation of odors. These two processing do not need to be performed consecutively, and each may be performed at any desired timing.

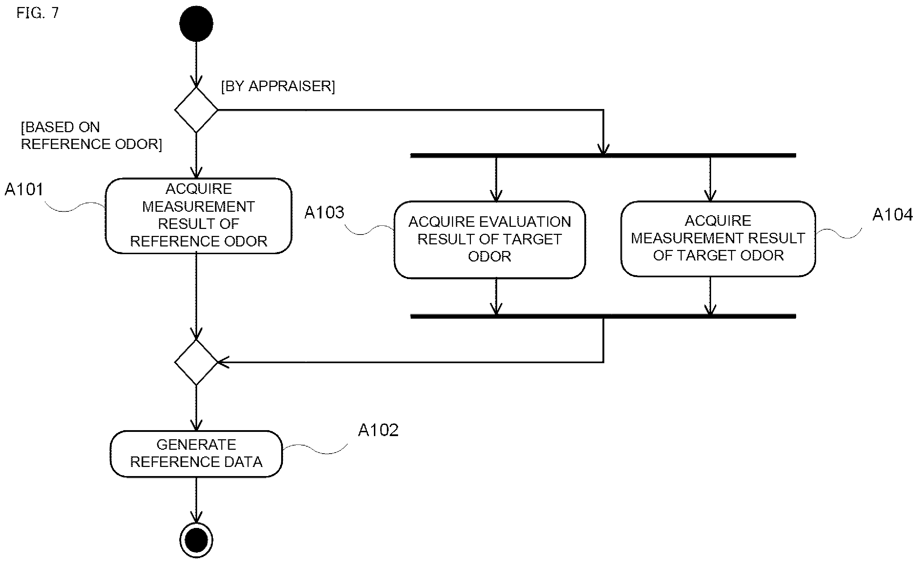

First, the flow of generating reference data will be described. The reference data is odor data based on a plurality of predetermined reference odors. FIG. 7 is an activity diagram showing a flow of generating reference data. In the case where the reference data is generated by using the reference odors, the second acquisition step acquires a set of a plurality of odor data detected from each of the reference odors by the sensor (A101). The sensor is provided in the detection apparatus 4 or the detection apparatus 5. Furthermore, the reference odors do not represent odor components such as ammonia, but are odors that indicate an article or its condition, and usually includes a plurality of odor components.

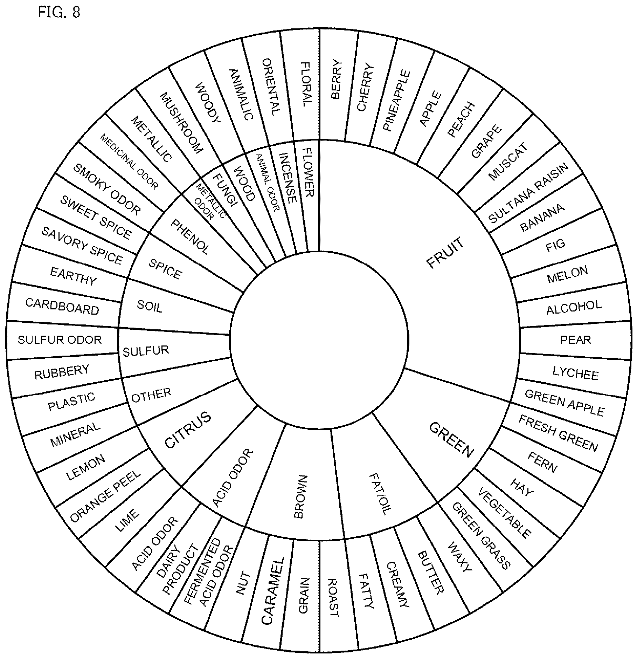

The reference odors are not defined at an element level, but rather define the features of odor quality of each odor without excess or deficiency and with minimal overlap, depending on the target product, etc. For example, the reference odors for sake are berry, cherry, pineapple, apple, peach, grape, muscat, sultana raisin, banana, fig, melon, alcohol, pear, lychee, green apple, fresh green, fern, hay, vegetable, green grass, waxy, butter, creamy, fatty, roast, grain, caramel, nut, fermented acid odor, dairy product, acid odor, lime, orange peel, lemon, mineral, plastic, rubbery, sulfur odor, cardboard, earthy, savory spice, sweet spice, smoky odor, medicinal odor, metallic, mushroom, woody, animalic, oriental, floral, etc. In addition, it is desirable that the reference data be based on commercially available reference odors or existing reference odors. There are commercially available reference odors corresponding to the aromas of, for example, wine, beer, whiskey, sake, brandy, etc. The names of the reference odors can be classified according to names such as fruit, green, fat/oil, brown, acid odor, citrus, other, sulfur, soil, spice, phenol, metallic odor, fungi, wood, animal odor, incense, flower, etc. The reference odors can be represented, for example, as an aroma wheel shown in FIG. 8. FIG. 8 shows an example of an aroma wheel.

Next, the generation step generates reference data based on the features of the odor data acquired in the second acquisition step (A102). The generation of the reference data is performed, for example, by learning the features of each reference odor by a machine learning, and by using the learned model as the reference data.

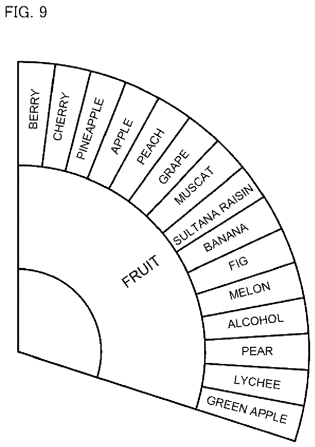

Furthermore, in the case where the reference data is generated by an evaluation by a person, for example, an appraiser, the second acquisition step acquires odor data detected from the reference target by the sensor and an evaluation of the reference target (A103). The reference target may be any item. However, in the case where the reference data for sake is generated, any sake should be the reference target, and in the case where the reference data for wine is generated, any wine should be the reference target, and so on. It is desirable that the reference target be an item that corresponds to the reference data to be generated. The sensor is provided in the detection apparatus 4 or the detection apparatus 5. The evaluation shows the odor of the reference target by the intensity of each of the reference odors. Note that in the case where the reference data is generated by an evaluation by an appraiser, the appraiser does not need to evaluate all of the reference odors shown in FIG. 8, and as shown in FIG. 9, the appraiser may evaluate only the reference odors classified as fruit among the reference odors shown in FIG. 8. FIG. 9 shows an example of reference odors used for evaluation by an appraiser.

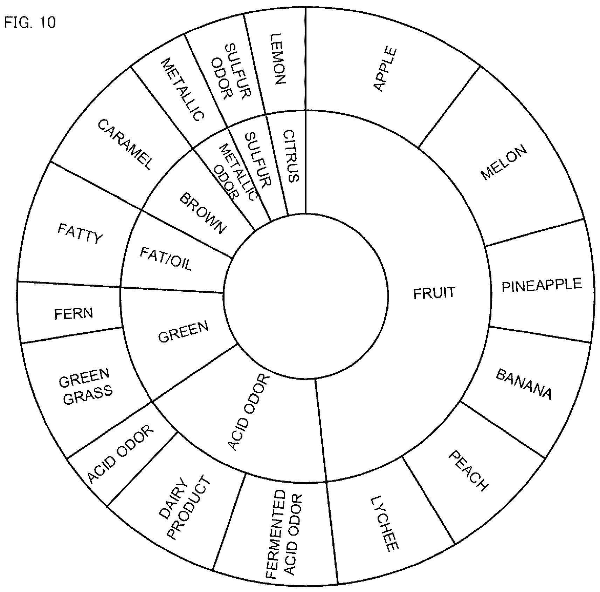

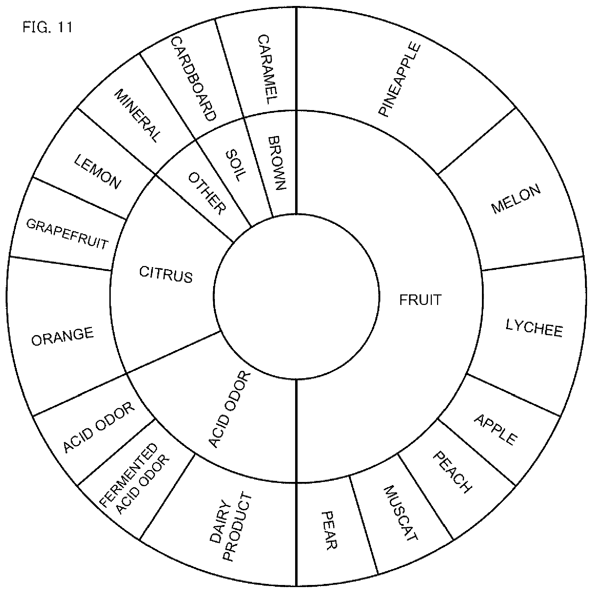

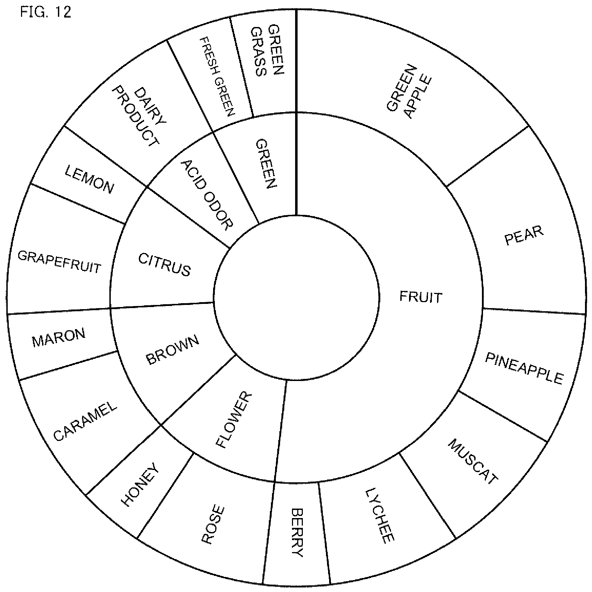

FIGs. 10 to 12 show examples of evaluations by an appraiser. In each of these drawings, a different brand of sake is used as the reference target, and each intensity of the reference odors is shown as the proportion of the area in a circle.

Next, the generation step generates the reference data based on the odor data acquired in the second acquisition step and the evaluation (A104). The generation of the reference data is performed, for example, by learning the relationship between the odor data of the reference target and the intensity of each reference odor by a machine learning, and using the learned model as the reference data.

By the way, the reference data can include a change over time of the odor of a reference object. Here, a change over time of an odor will be explained. For example, the odor and taste of wine and fragrances, etc. are not constant and change due to differences in the volatilization time of each substance. Thus, these cannot be expressed by the odor data that shows the odor at a certain point in time. Thus, by including time-dependent changes in the odor in the reference data, such changes can also be reflected in the visualization, thereby broadening the range of odor expression. The change over time of the odor is not limited to volatilization of substances but may also be due to the progress of fermentation or decomposition. Thus, an odor can also be used to manage fermentation and food. If these changes in odor can be visualized, more people can be involved in these tasks. A set of odor data including a change over time also includes changes in odor associated with human actions, for example, when a person drinks wine that has been poured into a glass, the changes between the state in which the glass is full of wine and the state in which the glass is empty.

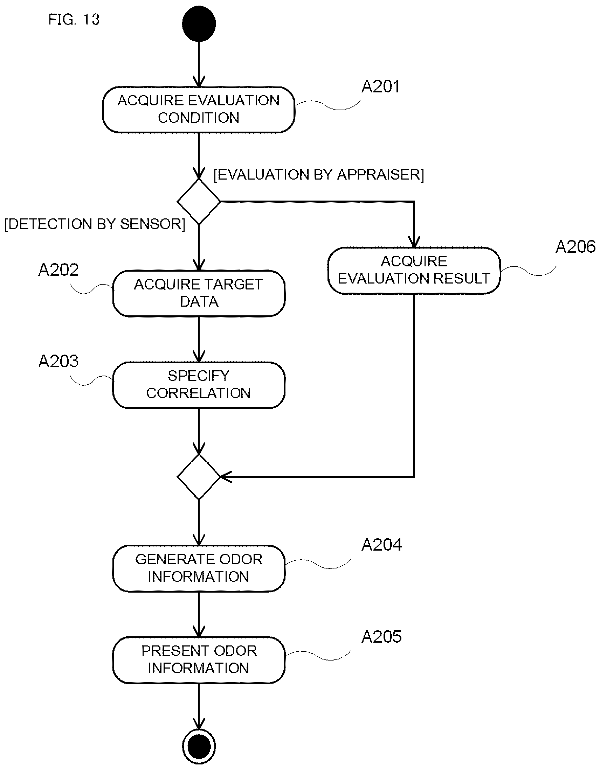

Next, the evaluation of odors based on the reference data and the presentation of odor information will be described. FIG. 13 is an activity diagram showing a flow of presenting odor information. First, at the time of presenting odor information, the evaluation conditions are acquired as the first acquisition step (A201). The evaluation conditions are the type of an evaluation target and a designation of a target person to whom the odor information is presented. For example, "presenting an evaluation of wine to developers" or "presenting an evaluation of sake to consumers" are equivalent to the evaluation conditions. It should be noted that it is also possible to omit acquiring the evaluation conditions.

Next, the first acquisition step acquires target data (A202). The target data is odor data detected from the evaluation target by the sensor, and the sensor is provided in the detection apparatus 4 or the detection apparatus 5. Then, the specification step specifies the correlation between the target data and at least a part of the reference data (A203). Specifically, the proportion of each of the reference odors of the reference data included in the target data is specified. The reason for specifying the correlation with at least a part of the reference data is that the number of required reference data differs depending on the presentation target of the odor information. For example, correlations with all reference odors are specified for developers, and correlations with a part of reference odors such as fruit odors are specified for consumers. In other words, the specification step specifies a correlation between the target data and the reference data corresponding to the presentation target of the odor information among the reference data. In addition, there are a plurality of types of reference data, such as for wine and for sake. Thus, among these, the reference data corresponding to the evaluation target is to be used. That is, the specification step specifies a correlation between the target data and the reference data corresponding to the evaluation target among the reference data.

Specifically, the correlation between the target data and the reference data is to specify the proportion of each of the reference odors of the reference data included in the target data. In the case where the reference data includes a change over time of the odor of a reference object, the specification step compares the target data and the reference data and specifies a reference object similar to the target data.

Next, the presentation step generates odor information (A204) and presents the generated odor information (A205). The odor information is information that visualizes the target data based on the correlation specified in the specification step, using the reference data that has specified the correlation as an index. Specific examples of the odor information will be described later.

In addition, the information processing apparatus 1 can also acquire evaluation results by a person such as an appraiser or the like as a third acquisition step (A206), generate odor information as the presentation step (A204), and present the generated odor information (A205).

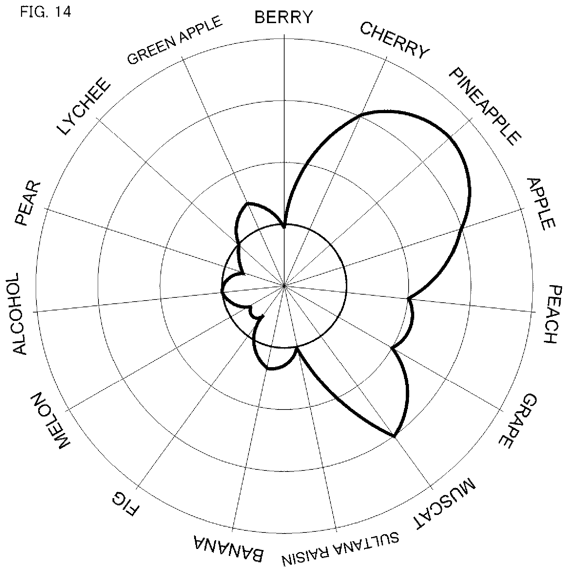

Here, several examples of presentation of odor information are described. FIG. 14 shows an example in which odor information is presented by a radar chart. In this drawing, the odor information is presented by a radar chart showing the intensity of the reference odors using the reference odors as an index.

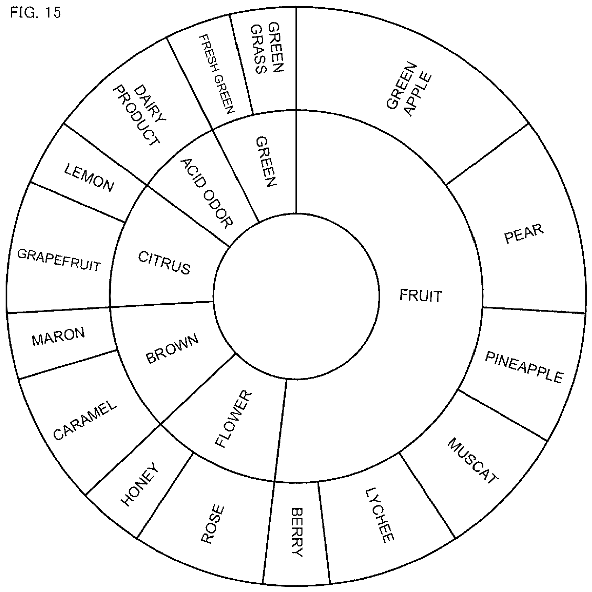

FIG. 15 shows an example in which odor information is presented using a circle. In this drawing, the odor information is presented as a set of divided circles, which are obtained by dividing a circle according to the intensity of the reference odors.

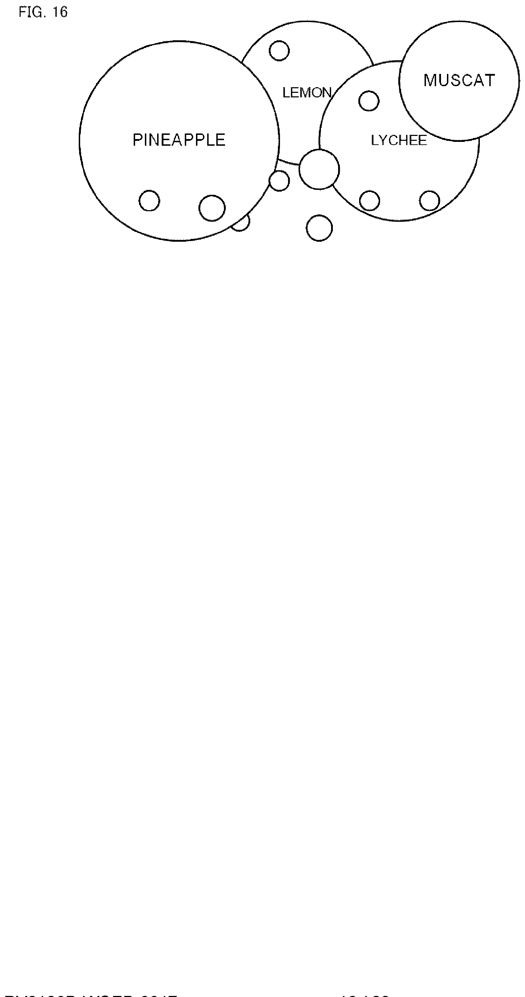

FIG. 16 shows an example in which odor information is presented using objects. In the example shown in this drawing, the odor information is an image in which objects of colors defined for each reference odor are arranged. In this case, the size of each object may differ depending on the intensity, proportion, etc. of the reference odor contained in the evaluation target. The color shade of each object may differ depending on the intensity, proportion, etc. of the reference odor contained in the evaluation target. Furthermore, the odor information may be shown as the number of objects according to the intensity, proportion, etc. of the reference odors contained in the evaluation target. For example, a picturesque image such as a pointillist painting may be presented.

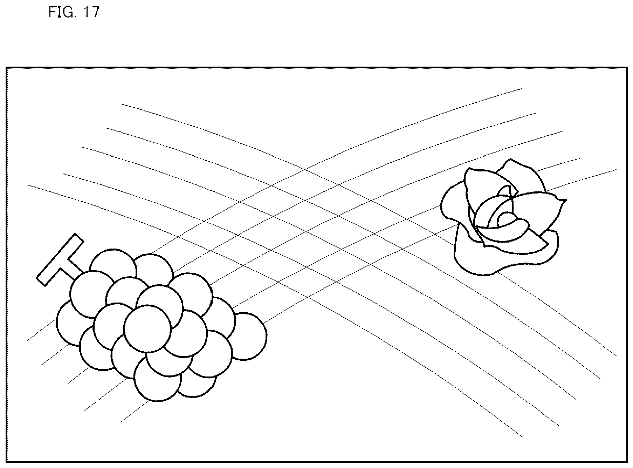

FIG. 17 shows an example in which odor information is presented using an illustration image. In the example shown in this drawing, in the case where the target data includes a specific pattern of a combination of the reference odors, the odor information includes an illustration image corresponding to the specific pattern. In this case, the background of the illustration image may be a picturesque image described above. In addition, a photographed image may also be used instead of the illustration images. In the example shown in this drawing, the target data includes a pattern similar to the odor of "rose" and "grape", and thus the images of "rose" and "grape" are included in the odor information.

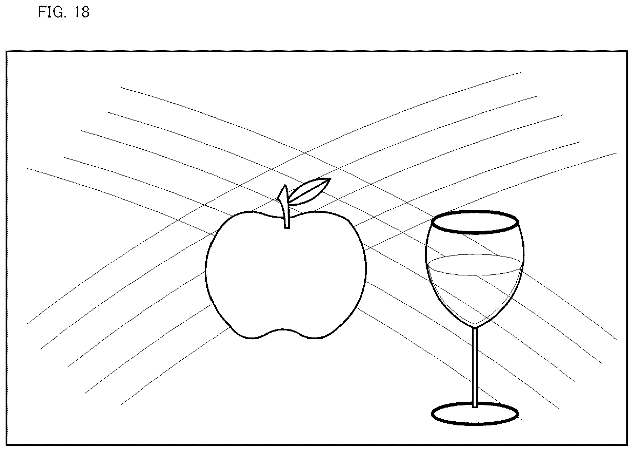

FIG.18 to FIG.20 show examples of presentation information that visualizes a change over time of an odor. In these drawings, the odor information includes information that visualizes the change over time of the odor of the reference object. Here, a change over time of an odor will be explained. For example, the odor and taste of wine and fragrances, etc. are not constant and change due to differences in the volatilization time of each substance. Thus, these cannot be expressed by the odor data that shows the odor at a certain point in time. Thus, by including time-dependent changes in the odor in the reference data, such changes can also be reflected in the visualization, thereby broadening the range of odor expression. The change over time of the odor is not limited to volatilization of substances but may also be due to the progress of fermentation or decomposition. Thus, an odor can also be used to manage fermentation and food. If these changes in odor can be visualized, more people can be involved in these tasks. A set of odor data including a change over time also includes changes in odor associated with human actions, for example, when a person drinks wine that has been poured into a glass, the changes between the state in which the glass is full of wine and the state in which the glass is empty.

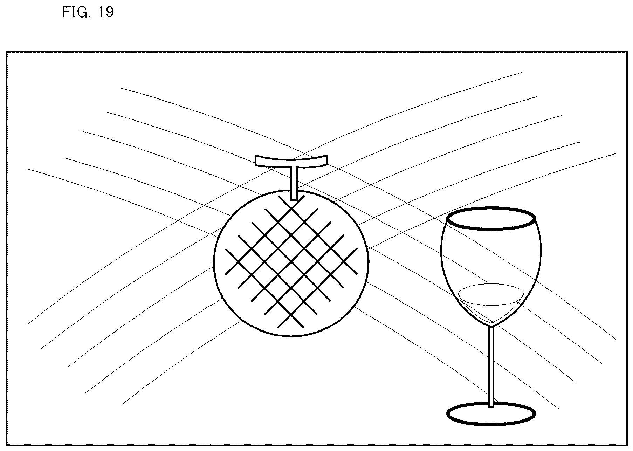

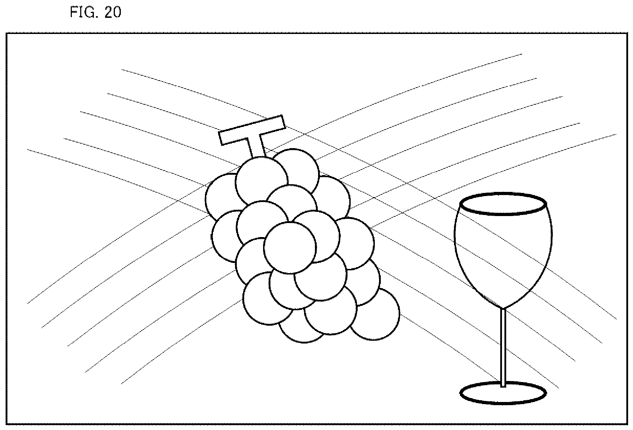

When visualizing a change over time of an odor, the odor information is represented as a slide image or a moving image. For example, the odor information shown in FIG. 18 indicates an odor associated with "apple" in a state where the glass is full of wine, and the odor information shown in FIG. 19 indicates an odor associated with "melon" in a state where only a small amount of wine is left in the glass. In addition, the odor information shown in FIG. 20 indicates an odor associated with "muscat" in a state where the glass is empty. The odor information does not necessarily correspond directly to an odor, but may include information associated with the odor, such as a landscape painting, a landscape photograph, etc.

### 8. Others

The present disclosure may be provided in each of the following aspects.
(1) An information processing method executed by an information processing system, comprising: a first acquisition step of acquiring target data that is odor data detected from an evaluation target by a sensor; a specification step of specifying a correlation between the target data and at least a part of reference data that is odor data based on a plurality of predetermined reference odors; and a presentation step of presenting odor information that is information visualizing the target data based on the correlation, using the reference data that has specified the correlation as an index.
(2) The information processing method according to (1), further comprising: a second acquisition step of acquiring a set of a plurality of odor data detected from each of the reference odors by a sensor; and a generation step of generating the reference data based on features of odor data acquired in the second acquisition step.
(3) The information processing method according to (1), further comprising: a second acquisition step of acquiring odor data detected from a reference target by a sensor and an evaluation of the reference target, the evaluation showing an odor of the reference target by intensity of each of the reference odors, and a generation step of generating the reference data based on odor data acquired in the second acquisition step and the evaluation.
(4) The information processing method according to any one of (1) to (3), wherein: the reference odors include a plurality of odor components.
(5) The information processing method according to any one of (1) to (4), wherein: the specification step specifies a correlation between the target data and reference data corresponding to a presentation target of the odor information among the reference data.
(6) The information processing method according to any one of (1) to (5), wherein: the specification step specifies a correlation between the target data and reference data corresponding to the evaluation target among the reference data.
(7) The information processing method according to any one of (1) to (6), wherein: the odor information is a radar chart showing intensity of the reference odors using the reference odors as an index.
(8) The information processing method according to any one of (1) to (6), wherein: the odor information is a set of divided circles obtained by dividing a circle according to intensity of the reference odors.
(9) The information processing method according to any one of (1) to (7), wherein: the odor information is an image in which objects of colors defined for each of the reference odors are arranged.
(10) The information processing method according to (9), wherein: a size of each of the objects differs depending on intensity of the reference odors contained in the evaluation target.
(11) The information processing method according to (9) or (10), wherein: a color shade of each of the objects differs depending on intensity of the reference odors contained in the evaluation target.
(12) The information processing method according to any one of (8) to (11), wherein: in a case where the target data includes a specific pattern of a combination of the reference odors, the odor information includes an image corresponding to the specific pattern.
(13) The information processing method according to any one of (1) to (12), wherein: the reference data includes a change over time of an odor of a reference object, the specification step compares the target data and the reference data and specifies a reference object similar to the target data, and the odor information includes information that visualizes the change over time of the odor of the reference object..
(14) The information processing method according to (13), wherein: the odor information is a slide image or a moving image.
(15) An information processing system comprising at least one device, comprising: at least one processor configured to execute a program so that each step of the information processing method according to any one of (1) to (14) is executed.
(16) An Information processing apparatus, comprising: at least one processor configured to execute a program so that each step of the information processing method according to any one of (1) to (14) is performed.
(17) A program, configured to allow at least one computer to execute each step of the information processing method according to any one of (1) to (14).

Of course, the present disclosure is not limited to the above aspects.

### Reference Signs List

1: Information processing apparatus
2: Network
3: Information processing terminal
4: Detection apparatus
5: Detection apparatus
11: Processing unit
12: Storage unit
13: Temporary storage unit
14: External apparatus connection unit
15: Communication unit
16: Communication bus
21: Processing unit
31: Processing unit
32: Storage unit
33: Temporary storage unit
34: External apparatus connection unit
35: Communication unit
36: Input unit
37: Display unit
38: Communication bus
41: Connection unit
42: Controller
43: Detection unit
51: Communication unit
52: Controller
53: Detection unit
54: Input unit
55: Display unit
100: System
101: Acquisition unit
102: Generation unit
103: Storage unit
104: Specification unit
105: Presentation unit

## Claims

1. An information processing method executed by an information processing system, comprising:
a first acquisition step of acquiring target data that is odor data detected from an evaluation target by a sensor;
a specification step of specifying a correlation between the target data and at least a part of reference data that is odor data based on a plurality of predetermined reference odors; and
a presentation step of presenting odor information that is information visualizing the target data based on the correlation, using the reference data that has specified the correlation as an index.

2. The information processing method according to claim 1, further comprising:
a second acquisition step of acquiring a set of a plurality of odor data detected from each of the reference odors by a sensor; and
a generation step of generating the reference data based on features of odor data acquired in the second acquisition step.

3. The information processing method according to claim 1, further comprising:
a second acquisition step of acquiring odor data detected from a reference target by a sensor and an evaluation of the reference target, the evaluation showing an odor of the reference target by intensity of each of the reference odors, and
a generation step of generating the reference data based on odor data acquired in the second acquisition step and the evaluation.

4. The information processing method according to any one of claims 1 to 3, wherein:
the reference odors include a plurality of odor components.

5. The information processing method according to any one of claims 1 to 4, wherein:
the specification step specifies a correlation between the target data and reference data corresponding to a presentation target of the odor information among the reference data.

6. The information processing method according to any one of claims 1 to 5, wherein:
the specification step specifies a correlation between the target data and reference data corresponding to the evaluation target among the reference data.

7. The information processing method according to any one of claims 1 to 6, wherein:
the odor information is a radar chart showing intensity of the reference odors using the reference odors as an index.

8. The information processing method according to any one of claims 1 to 6, wherein:
the odor information is a set of divided circles obtained by dividing a circle according to intensity of the reference odors.

9. The information processing method according to any one of claims 1 to 7, wherein:
the odor information is an image in which objects of colors defined for each of the reference odors are arranged.

10. The information processing method according to claim 9, wherein:
a size of each of the objects differs depending on intensity of the reference odors contained in the evaluation target.

11. The information processing method according to claim 9 or 10, wherein:
a color shade of each of the objects differs depending on intensity of the reference odors contained in the evaluation target.

12. The information processing method according to any one of claims 8 to 11, wherein:
in a case where the target data includes a specific pattern of a combination of the reference odors, the odor information includes an image corresponding to the specific pattern.

13. The information processing method according to any one of claims 1 to 12, wherein:
the reference data includes a change over time of an odor of a reference object,
the specification step compares the target data and the reference data and specifies a reference object similar to the target data, and
the odor information includes information that visualizes the change over time of the odor of the reference object.

14. The information processing method according to claim 13, wherein:
the odor information is a slide image or a moving image.

15. An information processing system comprising at least one device, comprising:
at least one processor configured to execute a program so that each step of the information processing method according to any one of claims 1 to 14 is executed.

16. An Information processing apparatus, comprising:
at least one processor configured to execute a program so that each step of the information processing method according to any one of claims 1 to 14 is performed.

17. A program, configured to allow at least one computer to execute each step of the information processing method according to any one of claims 1 to 14.
